# EUROPEAN PATENT APPLICATION

(11) **EP 1 698 703 A1**
(43) Date of publication of application: **06.09.2006**
(21) Application number: 04799915.6
(22) Date of filing: 26.11.2004
(51) Int. Cl.: C12P 21/06, C12N 1/20, C12R 1/01, C12R 1/38

(54) **PROCESS FOR PRODUCING DIPEPTIDE**

(30) Priority: 28.11.2003 JP 2003398810
(71) Applicant: KYOWA HAKKO KOGYO CO., LTD., Tokyo 100-8185 (JP)
(72) Inventor: IKEDA, Hajime ,c/o Technical Research Laboratories, Yamaguchi 747-8522 (JP); ADACHI, ,c/o Technical Research Laboratories, Yamaguchi 747-8522 (JP); YONETANI, Yoshiyuki,c/o BoiFrontier Laboratories, Machida-shi Tokyo 194-8533 (JP); HASHIMOTO, Shin-ichi,c/o Technical Research Lab., Yamaguchi 747-8522 (JP); YAGASAKI, Makoto,c/o Technical Research Laboratori, Yamaguchi 747-8522 (JP)
(74) Representative: Vossius & Partner
(86) International application number: PCT/JP2004/017980
(87) International publication number: WO 2005/052177

(57) **Abstract**

The present invention provides a process for producing a dipeptide which comprises: allowing an enzyme source and a diketopiperazine wherein one or two kinds of α-amino acids are condensed with each other to be present in an aqueous medium, said enzyme source being a culture of a microorganism having the ability to produce a dipeptide from a diketopiperazine wherein two kinds of α-amino acids are condensed with each other or a treated matter of the culture; allowing the dipeptide to form and accumulate in the aqueous medium; and recovering the dipeptide from the aqueous medium (provided that the case in which the diketopiperazine is a diketopiperazine wherein aspartic acid and phenylalanine are condensed with each other and the dipeptide is aspartylphenylalanine is excluded).

## Description

### Technical Field

The present invention relates to a microorganism having the ability to produce a dipeptide from a diketopiperazine wherein two kinds of α-amino acids are condensed with each other, and a process for producing a dipeptide using the microorganism.

### Background Art

Known methods for producing dipeptides include extraction from natural products, chemical synthesis and enzymatic methods. Extraction from natural products can be used only for producing limited kinds of dipeptides, and the productivity is low because the contents of desired dipeptides in natural products are low. In the synthesis of dipeptides by the chemical synthesis methods, operations such as introduction and removal of protective groups for functional groups are necessary, and racemates are also formed. The chemical synthesis methods are thus considered to be disadvantageous in respect of cost and efficiency. They are unfavorable also from the viewpoint of environmental hygiene because of the use of large amounts of organic solvents and the like.

As to the synthesis of dipeptides by the enzymatic methods, the following methods are known: a method utilizing reverse reaction of protease [J. Biol. Chem., 119, 707-720 (1937)]; methods utilizing thermostable aminoacyl t-RNA synthetase (Japanese Published Unexamined Patent Application No. 146539/83, Japanese Published Unexamined Patent Application No. 209991/83, Japanese Published Unexamined Patent Application No. 209992/83 and Japanese Published Unexamined Patent Application No. 106298/84); and methods utilizing non-ribosomal peptide synthetase (hereinafter referred to as NRPS) [Chem. Biol., 7, 373-384 (2000); FEBS Lett., 498, 42-45 (2001); US Patent No. 5795738 and US Patent No. 5652116].

However, the method utilizing reverse reaction of protease requires introduction and removal of protective groups for functional groups of amino acids used as substrates, which causes difficulties in raising the efficiency of peptide-forming reaction and in preventing peptidolytic reaction. The methods utilizing thermostable aminoacyl t-RNA synthetase have the defects that the expression of the enzyme and the prevention of reactions forming by-products are difficult. The methods utilizing NRPS are inefficient in that the expression of the enzyme by recombinant DNA techniques is difficult because the enzyme molecule is huge, and in that the supply of coenzyme 4'-phosphopantetheine is necessary.

As the enzyme having the ability to produce a dipeptide from a diketopiperazine wherein two kinds of α-amino acids are condensed with each other, an enzyme having the activity to form aspartylphenylalanine from a diketopiperazine wherein aspartic acid and phenylalanine are condensed with each other is known [New Frontiers in Screening for Microbial Biocatalysts, p. 201-210, Elsevier, Amsterdam (1998) and Japanese Published Unexamined Patent Application No. 208297/87]. Also known is a process for producing aspartylphenylalanine using, as an enzyme source, cells of a microorganism which produces this enzyme (Japanese Published Examined Patent Application No. 22238/96). However, there has been no report about the activity of the enzyme to form dipeptides from diketopiperazines other than the diketopiperazine wherein aspartic acid and phenylalanine are condensed with each other, or a process for producing dipeptides other than aspartylphenylalanine by using, as an enzyme source, cells of a microorganism which produces the enzyme.

There is also a report about an enzyme having the activity to form glycylglycine from a diketopiperazine wherein two glycines are condensed with each other [Agric. Biol. Chem., 49, 1567-1572 (1985)], while it is not known that the enzyme has the activity to form a dipeptide from a diketopiperazine wherein two kinds of α-amino acids are condensed with each other.

A lot of microorganisms are known to have the ability to decompose various kinds of diketopiperazines [J. Biosci. Bioeng., 89, 602-605 (2000); New Frontiers in Screening for Microbial Biocatalysts, p. 167-171, Elsevier, Amsterdam (1998); J. Ferment. Bioeng., 83, 386-388 (1997) and Journal of Bioscience and Bioengineering, 79, 71-77 (2001)], but none of the microorganisms is known to form a dipeptide.

### Disclosure of the Invention

An object of the present invention is to provide a microorganism having the ability to produce a dipeptide from a diketopiperazine and a process for producing a dipeptide utilizing the microorganism.

The present invention relates to the following (1) to (12).
(1) A process for producing a dipeptide, which comprises:
   allowing an enzyme source and a diketopiperazine wherein one or two kinds of α-amino acids or derivatives thereof are condensed with each other to be present in an aqueous medium, said enzyme source being a culture of a microorganism having the ability to produce a dipeptide from a diketopiperazine wherein two kinds of α-amino acids are condensed with each other or a treated matter of the culture;
   allowing the dipeptide to form and accumulate in the aqueous medium; and
   recovering the dipeptide from the aqueous medium (provided that the case in which the diketopiperazine is a diketopiperazine wherein aspartic acid and
   phenylalanine are condensed with each other and the dipeptide is aspartylphenylalanine is excluded).
(2) The process according to the above (1), wherein the microorganism having the ability to produce a dipeptide from a diketopiperazine wherein two kinds of α-amino acids are condensed with each other is a microorganism which produces dipeptides in which the proportion of one kind of dipeptide is 70% or more.
(3) The process according to the above (1) or (2), wherein the microorganism having the ability to produce a dipeptide from a diketopiperazine wherein two kinds of α-amino acids are condensed with each other is a microorganism obtained by a method comprising:
   [1] the step of culturing test microorganisms using a medium comprising a diketopiperazine wherein two kinds of α-amino acids are condensed with each other as the sole carbon source or nitrogen source;
   [2] the step of selecting microorganisms which are recognized to grow in the above step [1]; and
   [3] the step of selecting a microorganism which forms and accumulates a dipeptide in an aqueous medium when the diketopiperazine used in the above step [1] and the microorganisms selected in the above step [2] are allowed to be present in the aqueous medium.
(4) The process according to the above (2), wherein the microorganism having the ability to produce a dipeptide from a diketopiperazine wherein two kinds of α-amino acids are condensed with each other is a microorganism obtained by a method comprising:
   [1] the step of culturing test microorganisms using a medium comprising a diketopiperazine wherein two kinds of α-amino acids are condensed with each other as the sole carbon source or nitrogen source;
   [2] the step of selecting microorganisms which are recognized to grow in the above step [1]; and
   [3] the step of selecting a microorganism which forms and accumulates dipeptides in an aqueous medium, the proportion of one kind of dipeptide in the dipeptides formed and accumulated being 70% or more, when the diketopiperazine used in the above step [1] and the microorganisms selected in the above step [2] are allowed to be present in the aqueous medium.
(5) The process according to any one of the above (1) to (4), wherein the microorganism having the ability to produce a dipeptide from a diketopiperazine wherein two kinds of α-amino acids are condensed with each other is a microorganism belonging to the genus Microbacterium, Sinorhizobium or Pseudomonas.
(6) The process according to the above (5), wherein the microorganism belonging to the genus Microbacterium is Microbacterium luteolum.
(7) A process for producing a dipeptide, which comprises:
   allowing an enzyme source and a diketopiperazine wherein one or two kinds of α-amino acids or derivatives thereof are condensed with each other to be present in an aqueous medium, said enzyme source being a culture of a microorganism belonging to the genus Microbacterium, Sinorhizobium or Pseudomonas having the ability to produce a dipeptide from a diketopiperazine wherein two kinds of α-amino acids are condensed with each other or a treated matter of the culture;
   allowing the dipeptide to form and accumulate in the aqueous medium; and
   recovering the dipeptide from the aqueous medium.
(8) The process according to the above (7), wherein the microorganism belonging to the genus Microbacterium is Microbacterium luteolum.
(9) The process according to any one of the above (1) to (8), wherein the α-amino acid is an α-amino acid selected from the group consisting of alanine, glutamine, glutamic acid, glycine, valine, leucine, isoleucine, proline, phenylalanine, tryptophan, methionine, serine, threonine, cysteine, asparagine, tyrosine, lysine, arginine, histidine, aspartic acid and ornithine.
(10) The process according to any one of the above (1) to (9), wherein the two kinds of α-amino acids are alanine and glutamine, and the dipeptide is alanylglutamine.
(11) The process according to any one of the above (1) to (10), wherein the treated matter of the culture is concentrated culture, dried culture, cells obtained by centrifuging the culture, or a product obtained by subjecting the cells to drying, freeze-drying, treatment with a surfactant, treatment with a solvent, enzymatic treatment, immobilization, mechanical friction or ultrasonication.
(12) A microorganism having the ability to produce a dipeptide from a diketopiperazine wherein two kinds of α-amino acids are condensed with each other which is selected from the group consisting of
   Microbacterium luteolum No. 93 (FERM BP-08513),
   Microbacterium sp. No. 119 (FERM BP-08514),
   Sinorhizobium sp. No. 1 (FERM BP-08509),
   Sinorhizobium sp. No. 164 (FERM BP-08510),
   Pseudomonas sp. No. 107 (FERM BP-08511) and
   Pseudomonas sp. No. 108 (FERM BP-08512).

The present invention is described in detail below.

### 1. Diketopiperazines To Be Used in the Process of the Present Invention

The diketopiperazines wherein one or two kinds of α-amino acids or derivatives thereof are condensed with each other to be used in the present invention include any diketopiperazines wherein one or two different kinds of α-amino acids or derivatives thereof are condensed with each other.

There is no specific restriction as to the α-amino acids so far as they can be condensed with each other to form the diketopiperazine structure, and preferred examples of α-amino acids include L-α-amino acids, D-α-amino acids, glycine and derivatives thereof, more preferably L-α-amino acids, glycine and derivatives thereof.

Preferred examples of L-α-amino acids and D-α-amino acids are L- and D-forms of alanine, glutamine, glutamic acid, valine, leucine, isoleucine, proline, phenylalanine, tryptophan, methionine, serine, threonine, cysteine, asparagine, tyrosine, lysine, arginine, histidine, aspartic acid and ornithine.

There is no specific restriction as to the derivatives of α-amino acids so far as they can be condensed with each other to form the diketopiperazine structure, and preferred derivatives include N-methyl amino acids, specifically, N-methyl-alanine, N-methyl-glutamine, N-methyl-glutamic acid, N-methyl-glycine, N-methyl-valine, N-methyl-leucine, N-methyl-isoleucine, N-methyl-proline, N-methyl-phenylalanine, N-methyl-tryptophan, N-methyl-methionine, N-methyl-serine, N-methyl-threonine, N-methyl-cysteine, N-methyl-asparagine, N-methyl-tyrosine, N-methyl-lysine, N-methyl-arginine, N-methyl-histidine, N-methyl-aspartic acid and N-methyl-ornithine.

There is no specific restriction as to the derivatives of L-α-amino acids, D-α-amino acids and glycine so far as they can be condensed with each other to form the diketopiperazine structure, and preferred derivatives include hydroxyamino acids, specifically, β-hydroxyglutamine, β-hydroxyglutamic acid, γ-hydroxyglutamic acid, α-hydroxyglycine, β-hydroxyvaline, γ-hydroxyvaline, β-hydroxyleucine, γ-hydroxyleucine, δ-hydroxyleucine, β-hydroxyisoleucine, γ-hydroxyisoleucine, 3-hydroxyproline, 4-hydroxyproline, β-hydroxyphenylalanine, 3,4-dihydroxyphenylalanine, 2,4,5-trihydroxyphenylalanine, β-hydroxytryptophan, 5-hydroxytryptophan, α-hydroxymethionine, β-hydroxyserine, γ-hydroxythreonine, S-hydroxycysteine, β-hydroxyasparagine, β-hydroxytyrosine, β-hydroxylysine, γ-hydroxylysine, δ-hydroxylysine, N-hydroxylysine, β-hydroxyarginine, δ-hydroxyarginine, N-hydroxyarginine, β-hydroxyhistidine, β-hydroxyaspartic acid, β-hydroxyornithine, γ-hydroxyornithine and N-hydroxyornithine.

The methods for producing the above diketopiperazines include chemical synthesis methods [J. Comb. Chem., 3, 453-460 (2001); Tetrahedron, 58, 3297-3312 (2002), etc.], enzymatic methods [Chemistry Biology, 8, 997-1010 (2001); Chemistry Biology, 9, 1355-1364 (2002), etc.] and the like.

For example, production of a diketopiperazine wherein alanine and glutamine are condensed with each other [hereinafter referred to as cyclo(Ala-Gln)] can be carried out by the chemical synthesis method in the following manner.

Alanylglutamine (for example, Product G-1210, Bachem) is dissolved in a 2 mol/l aqueous solution of sodium hydroxide, and 1.5 equivalents of benzyloxycarbonyl chloride is added thereto to form benzyloxycarbonylated alanylglutamine (hereinafter referred to as Z-Ala-Gln). To the resulting solution is added concentrated hydrochloric acid to adjust the solution to pH 2, and the formed Z-Ala-Gln is obtained as crystals. The crystals are dissolved in an N,N-dimethylformamide-ethyl acetate solution (4:1), and 1 equivalent of N-hydroxysuccinimide and 1 equivalent of dicyclohexylcarbodiimide are added thereto to form succinimidized Z-Ala-Gln (hereinafter referred to as Z-Ala-Gln-ONSu). Then, the solution is concentrated under reduced pressure to obtain Z-Ala-Gln-ONSu as crystals, and the obtained crystals are suspended in a methanol-water solution (95:5). To the suspension is added palladium carbon to form cyclo(Ala-Gln), followed by concentration under reduced pressure to obtain cyclo(Ala-Gln) as crystals.

The chemical synthesis methods and the enzymatic methods, which do not require protection and deprotection of functional groups in amino acids, can efficiently produce diketopiperazines without depending on the structure of α-amino acids.

### 2. Microorganisms To Be Used in the Process of the Present Invention

The microorganisms having the ability to produce a dipeptide from a diketopiperazine wherein two kinds of α-amino acids are condensed with each other to be used in the process of the present invention may be any microorganisms having such ability, including microorganisms isolated from nature, mutant strains obtained by treating the microorganisms with drugs and ultraviolet irradiation, cell fusion strains and recombinant strains.

There is no specific restriction as to the two kinds of α-amino acids so far as they can be condensed with each other to form the diketopiperazine structure, and preferred examples include two different kinds of α-amino acids selected from the group consisting of L-α-amino acids, D-α-amino acids, glycine and derivatives thereof.

Preferred L-α-amino acids, D-α-amino acids, and derivatives of L-α-amino acids, D-α-amino acids and glycine include the amino acids and derivatives thereof mentioned in the above 1.

The microorganisms having the ability to produce a dipeptide from a diketopiperazine wherein two kinds of α-amino acids are condensed with each other to be used in the process of the present invention include microorganisms characterized in that the proportion of one kind of dipeptide in the dipeptides produced from a diketopiperazine wherein two kinds of α-amino acids are condensed with each other is preferably 70% or more, more preferably 80% or more, further preferably 90% or more, particularly preferably 95% or more, and most preferably 100%. However, microorganisms which produce the enzyme described in New Frontiers in Screening for Microbial Biocatalysts, p. 201-210, Elsevier, Amsterdam (1998) and Japanese Published Unexamined Patent Application No. 208297/87 are not included in the microorganisms of the present invention.

"The proportion of one kind of dipeptide in the dipeptides produced from a diketopiperazine wherein two kinds of α-amino acids are condensed with each other" refers to the proportion of A-B or B-A (two kinds of α-amino acids are referred to as A and B, respectively, and the dipeptides formed from a diketopiperazine wherein A and B are condensed with each other are referred to as A-B and B-A, respectively; "-" represents an amide bond) to the total amount of formed dipeptides (A-B+B-A).

The microorganisms to be used in the process of the present invention can be obtained, for example, in the following manner.

A soil sample (ca. 0.5 to 5 g) is suspended in sterile water, and the resulting suspension is gently shaken at room temperature for 10 minutes to 1 hour and allowed to stand for 1 to 30 minutes. The obtained supernatant (0.05 to 2 ml) is spread on an agar medium comprising a diketopiperazine wherein two kinds,of α-amino acids are condensed with each other as the sole carbon source or nitrogen source (hereinafter referred to as a DKP-containing agar medium) and cultured at 25 to 60°C for 1 to 5 days to form colonies.

There is no specific restriction as to the diketopiperazine used above so far as it is a diketopiperazine wherein two kinds of α-amino acids are condensed with each other, and preferred examples are diketopiperazines wherein L-α-amino acids are condensed, more preferably a diketopiperazine wherein L-alanine and L-glutamine are condensed with each other.

The DKP-containing agar medium used above may be any medium that comprises a diketopiperazine wherein two kinds of α-amino acids are condensed with each other as the sole carbon source or nitrogen source, and comprises a nitrogen source and inorganic salts when the diketopiperazine is used as the sole carbon source, or comprises a carbon source and inorganic salts when the diketopiperazine is used as the sole nitrogen source.

Examples of the carbon sources include carbohydrates such as glucose, fructose, sucrose, starch and starch hydrolyzate, organic acids such as acetic acid and propionic acid, and alcohols such as ethanol and propanol, which comprise no nitrogen.

Examples of the nitrogen sources include ammonia and ammonium salts of organic or inorganic acids such as ammonium chloride, ammonium sulfate, ammonium acetate and ammonium phosphate.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Then, the formed colonies are spread on the DKP-containing agar medium and an agar medium prepared by removing the diketopiperazine from the DKP-containing agar medium, and cultured at 25 to 60°C for 1 to 5 days. Microorganisms having the ability to assimilate the diketopiperazine can be obtained by selecting strains that grow on the DKP-containing agar medium and do not grow on the agar medium prepared by removing the diketopiperazine from the DKP-containing agar medium.

The strains obtained above are spread on a synthetic agar medium and cultured at 25 to 60°C for 1 to 5 days. Then, one platinum loop of each microorganism that grew on the agar medium is inoculated into a liquid medium comprising the above diketopiperazine and cultured with shaking at 25 to 60°C for 1 to 5 days.

After the completion of the culturing, the supernatant of each culture is analyzed to select strains giving a culture from which the diketopiperazine is not detected as the strains having the ability to decompose the diketopiperazine. Analysis of the culture supernatant may be carried out by any method capable of detecting the employed diketopiperazine, for example, a method utilizing high performance liquid chromatography.

The strains thus selected are spread on a synthetic agar medium and cultured at 25 to 60°C for 1 to 5 days. Then, one platinum loop of each strain is inoculated into a liquid medium comprising 1 to 5 g/l diketopiperazine and cultured with shaking at 25 to 60°C for 1 to 5 days. After the completion of the culturing, the culture is centrifuged and the collected cells are washed with saline or the like. The washed cells can be used in the following operations as such or after being frozen at - 80°C for storage and then thawed.

The cells, after being thawed at an ordinary temperature in the case of frozen cells, are suspended in a buffer to a concentration of 5 to 50 g/l in terms of wet cell weight to prepare a cell suspension. As the buffer, any buffers can be used, for example, Tris buffer and phosphate buffer.

A metal ion, a chelating agent or a dipeptide analogue may be added to the suspension in order to inhibit the activity to hydrolyze a dipeptide formed.

To the suspension is added the diketopiperazine to a concentration of 1 to 5 g/l to prepare a reaction mixture, and the mixture is gently shaken at 25 to 60°C for 1 to 24 hours. After the completion of the reaction, the reaction mixture is centrifuged, and the obtained supernatant is analyzed to detect the formation of a dipeptide. A microorganism having the ability to produce a dipeptide from the diketopiperazine can be obtained by selecting a strain that gives a reaction mixture in which the formation and accumulation of the dipeptide are detected.

The detection of the dipeptide may be carried out by any method that can detect the dipeptide, for example, a method in which the dipeptide in the supernatant is derivatized by 9-fluorenylmethoxycarbonyl (FMOC) and then analyzed by HPLC.

Examples of the microorganisms of the present invention obtained by the above method are those belonging to the genus Microbacterium, Sinorhizobium or Pseudomonas, and an example of the microorganisms belonging to the genus Microbacterium is Microbacterium luteolum.

More specific examples of the microorganisms of the present invention are Microbacterium luteolum No. 93, Microbacterium sp. No. 119, Sinorhizobium sp. No. 1, Sinorhizobium sp. No. 164, Pseudomonas sp. No. 107, Pseudomonas sp. No. 108, microorganisms obtained by serial subcultivation of these strains, and cell fusion strains and mutant strains prepared using these strains.

The above-mentioned strains are microorganisms newly isolated from the soil by the present inventors. The results of identification of the strains are shown below.

As a result of morphological observation of strains No. 93 and No. 119 isolated from the soil, they were both found to be Gram-positive rods. The 16S rDNA partial sequences of the respective strains were amplified and obtained using genomic DNAs prepared from strains No. 93 and No. 119 as templates, and using 16S rDNA universal primers 29f and 1492r described in Lett. Appl. Microbiol., 15, 210-213 (1992) as a set of primers. The PCR products were purified with ExoSap-IT (Pharmacia) to determine the nucleotide sequences.

Blast search was carried out using, as a query, the respective 16S rDNA partial sequences of strains No. 93 and No. 119 determined above, whereby it was confirmed that the 16S rDNAs of both strains had a high homology to the 16S rDNA of a microorganism belonging to the genus Microbacterium of the class Actinobacteria which is a Gram-positive bacterium. Then, phylogenetic analysis was carried out by obtaining the 16S rDNA sequences of related strains from the rRNA database. The sequences of strains No. 93 and No. 119 were aligned with the sequences of related strains obtained as a multi-alignment file to which the secondary structural information of an rRNA molecule was added, using the profile alignment menu of the multi-alignment preparing software clustalW (downloaded from the website of EMBL).

On the basis of the prepared alignment, the genetic distances among the strains based on Kimura's 2-parameter method were calculated using the dnadist program in the PHYLIP package (downloaded from the website of University of Washington, Felsenstein Lab), and the phylogenetic relation was inferred by the neighbor-joining method using the neighbor program (downloaded from the website of University of Washington, Felsenstein Lab). A phylogenetic tree was consructed using the NJplot program (downloaded from the website of Pole Bio-Informatique Lyonnais) and 100 data sets were generated using the seqboot program in the PHYLIP package to confirm the reliability of branches of the phylogenetic tree by the bootstrap analysis.

As a result, it was indicated that both of the strains are very closely related to Microbacterium luteolum, Microbacterium oxydans and Microbacterium liquefaciens of the genus Microbacterium. The sequence of strain No. 93 was analogous especially to the sequence of Microbacterium luteolum, and this clustering was supported by the bootstrap value of 93%. However, the phylogenetic distances among these four strains including the No. 93 strain are extremely short and it was difficult to carry out an accurate analysis of the phylogenetic relations within this group of strains.

In order to clarify the taxonomic relationship between these three strains and the No. 93 strain at species level, DNA/DNA hybridization test was carried out. As test strains, the No. 93 strain and standard strains of Microbacterium luteolum, Microbacterium oxydans and Microbacterium liquefaciens (IFO 1574, IFO 15586 and IFO 15037, respectively, Institute for Fermentation, Osaka) were used, and as a negative control, Corynebacterium glutamicum ATCC 13032 was used. DNAs which were obtained by extracting genomic DNAs from the respective strains by a known method and purifying them with hydroxyapatite were employed in the test.

As a result of DNA/DNA hybridization test, strain No. 93 showed a high DNA/DNA homology (97% or more) to the IFO 15074 strain which is a standard strain of Microbacterium luteolum. Strain No. 93 also showed a relatively high DNA/DNA homology to the standard strains of Microbacterium oxydans and Microbacterium liquefaciens which were also revealed to be closely related by the 16S rDNA phylogenetic analysis. However, its value was 50 to 60%, which is equal to the DNA/DNA homology observed between related strains of different species.

From the above results, strains No. 93 and No. 119 were identified as Microbacterium luteolum and Microbacterium sp., respectively.

As a result of morphological observation, strains No. 1 and No. 164 isolated from the soil were found to be Gram-negative rods. The nucleotide sequences of the 16S rDNA partial sequences of the respective strains were determined in the same manner as descried above, using, as templates, genomic DNAs prepared from strains No. 1 and No. 164, and then subjected to Blast search, whereby it was confirmed that the sequences have a high homology to the 16S rDNA sequence of a microorganism belonging to the genus Sinorhizobium of α-Proteobacteria. Then, the 16S rDNA sequences of related strains were obtained from the rRNA database and phylogenetic analysis was carried out in the same manner as described above. As a result, it was revealed that both of the strains belong to the same phylogenetic group as a strain belonging to the genus Sinorhizobium, and are very closely related to Sinorhizobium morelense and Ensifer adhaerens. The advice has been submitted to the Committee on Classification of Prokaryote, International Union of Microbiological Societies that the genus Ensifer should be treated as included in the genus Sinorhizobium [Int. J. Syst. Envol. Microbiol., 52, 2337 (2002)].

Based on the above phylogenetic analysis result and the opinion of the Committee on Classification of Prokaryotes, strains No. 1 and No. 164 were identified as Sinorhizobium sp.

As a result of morphological observation, strains No. 107 and No. 108 isolated from the soil were found to be Gram-negative rods. The nucleotide sequences of the 16S rDNA partial sequences of the respective strains were determined in the same manner as described above, using, as templates, genomic DNAs prepared from strains No. 1 and No. 164, and then subjected to Blast search, whereby it was confirmed that the sequences have a high homology to the 16S rDNA sequence of a microorganism belonging to the genus Pseudomonas of γ-Proteobacteria. Then, the 16S rDNA sequences of related strains were obtained from the rRNA database and phylogenetic analysis was carried out in the same manner as described above. As a result, it was revealed that both of the strains belong to the same phylogenetic group as a strain belonging to the genus Pseudomonas, and are very closely related to Pseudomonas graminis.

From the above phylogenetic analysis result, strains No. 107 and No. 108 were identified as Pseudomonas sp.

Microbacterium luteolum No. 93, Microbacterium sp. No. 119, Sinorhizobium sp. No. 1, Sinorhizobium sp. No. 164, Pseudomonas sp. No. 107 and Pseudomonas sp. No. 108 were deposited with International Patent Organism Depositary, National Institute of Advanced Industrial Science and Technology (AIST), Tsukuba Central 6, 1-1-1, Higashi, Tsukuba, Ibaraki 305-8566, Japan, on October 17, 2003 with accession numbers FERM BP-08513, FERM BP-08514, FERM BP-08509, FERM BP-08510, FERM BP-08511 and FERM BP-08512, respectively, under the Budapest Treaty.

### 3. Production Process of the Present Invention

The present invention relates to a process for producing a dipeptide which comprises allowing an enzyme source and a diketopiperazine wherein one or two kinds of α-amino acids or derivatives thereof are condensed with each other to be present in an aqueous medium, said enzyme source being a culture of a microorganism having the ability to produce a dipeptide from a diketopiperazine wherein two kinds of α-amino acids are condensed with each other or a treated matter of the culture, allowing the dipeptide to form and accumulate in the aqueous medium, and recovering the dipeptide from the aqueous medium. However, the process wherein the diketopiperazine is a diketopiperazine wherein aspartic acid and phenylalanine are condensed with each other and the dipeptide is aspartylphenylalanine is not included in the present invention. Preferably, the process wherein the diketopiperazine is a diketopiperazine wherein glycines are condensed with each other is not included in the present invention, either.

The present invention also relates to a process for producing a dipeptide which comprises allowing an enzyme source and a diketopiperazine wherein one or two kinds of α-amino acids or derivatives thereof are condensed with each other to be present in an aqueous medium, said enzyme source being a culture of a microorganism belonging to the genus Microbacterium, Sinorhizobium or Pseudomonas or a treated matter of the culture, allowing the dipeptide to form and accumulate in the aqueous medium, and recovering the dipeptide from the aqueous medium.

Culturing of the microorganism having the ability to produce a dipeptide from a diketopiperazine wherein two kinds of α-amino acids are condensed with each other, the microorganism belonging to the genus Microbacterium, the microorganism belonging to the genus Sinorhizobium and the microorganism belonging to the genus Pseudomonas can be carried out according to an ordinary method employed for culturing a microorganism.

Examples of the microorganism having the ability to produce a dipeptide from a diketopiperazine wherein two kinds of α-amino acids are condensed with each other, the microorganism belonging to the genus Microbacterium, the microorganism belonging to the genus Sinorhizobium and the microorganism belonging to the genus Pseudomonas include the microorganisms described in the above 2.

As the medium for culturing the microorganism, any of natural media and synthetic media can be used insofar as it is a medium suitable for efficient culturing of the microorganism which contains carbon sources, nitrogen sources, inorganic salts, etc. which can be assimilated by the microorganism.

As the carbon sources, any carbon sources that can be assimilated by the microorganism can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

Examples of the nitrogen sources include ammonia, ammonium salts of organic or inorganic acids such as ammonium chloride, ammonium sulfate, ammonium acetate and ammonium phosphate, nitrogen-containing compounds, peptone, meat extract, yeast extract, corn steep liquor, casein hydrolyzate, soybean cake, soybean cake hydrolyzate, and various fermented microbial cells and digested products thereof.

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 60°C, and the culturing period is usually 5 hours to 7 days. The pH is maintained at 4 to 10 during the culturing. The pH adjustment is carried out by using an organic or inorganic acid, an alkali solution, urea, calcium carbonate, ammonia, etc.

Examples of the treated matters of the culture include treated matters containing living cells such as concentrated culture, dried culture, cells obtained by centrifuging the culture, products obtained by subjecting the cells to drying, freeze-drying, treatment with a surfactant, treatment with a solvent, enzymatic treatment and immobilization, and treated matters containing crude enzyme extracts such as products obtained by subjecting the cells to mechanical friction and ultrasonication.

The enzyme source used in dipeptide-forming reaction is used at a concentration of 1 mU/l to 1000 U/l, preferably 10 mU/l to 100 U/l, one unit (U) being defined as the activity to form 1 mmol of dipeptide from a specific diketopiperazine at 30°C in one minute.

The diketopiperazine wherein one or two kinds of α-amino acids or derivatives thereof are condensed with each other used as a substrate is used at a concentration of 1 to 500 g/l.

The aqueous media used in the dipeptide-forming reaction include water, buffers such as phosphate buffer, carbonate buffer, acetate buffer, borate buffer, citrate buffer and Tris buffer, alcohols such as methanol and ethanol, esters such as ethyl acetate, ketones such as acetone, and amides such as acetamide. The culture of a microorganism used as the enzyme source can also be used as the aqueous medium.

In the dipeptide-forming reaction, a surfactant or an organic solvent may be added according to need. Any surfactant that promotes the formation of a dipeptide can be used. Suitable surfactants include nonionic surfactants such as polyoxyethylene octadecylamine (e.g., Nymeen S-215, NOF Corporation), cationic surfactants such as cetyltrimethylammonium bromide and alkyldimethylbenzylammonium chloride (e.g., Cation F2-40E, NOF Corporation), anionic surfactants such as lauroyl sarcosinate, and tertiary amines such as alkyldimethylamine (e.g., Tertiary Amine FB, NOF Corporation), which may be used alone or in combination. The surfactant is usually used at a concentration of 0.1 to 50 g/l. As the organic solvent, xylene, toluene, aliphatic alcohols, acetone, ethyl acetate, etc. may be used usually at a concentration of 0.1 to 50 ml/l.

The dipeptide-forming reaction is carried out in the aqueous medium at pH 5 to 10, preferably pH 6 to 9, at 20 to 60°C for 1 to 96 hours. If necessary, an inorganic salt such as cobalt chloride (CoCl₂), a chelating agent such as EDTA, a substance inhibiting dipeptide-decomposing activity such as bestatin, etc. can be added in order to inhibit decomposition of a dipeptide.

The dipeptide formed in the aqueous medium can be recovered by ordinary isolation and purification methods using activated carbon, ion-exchange resins, etc.

### 4. Microorganisms of the Present Invention

Examples of the microorganisms of the present invention are Microbacterium luteolum No. 93, (FERM BP-08513), Microbacterium sp. No. 119 (FERM BP-08514), Sinorhizobium sp. No. 1 (FERM BP-08509), Sinorhizobium sp. No. 164 (FERM BP-08510), Pseudomonas sp. No. 107 (FERM BP-08511) and Pseudomonas sp. No. 108 (FERM BP-08512).

Certain embodiments of the present invention are illustrated in the following examples. These examples are not to be construed as limiting the scope of the present invention.

The diketopiperazine wherein alanine and glutamine are condensed with each other [hereinafter referred to as cyclo(Ala-Gln)] used in the examples was prepared in the following manner.

Ala-Gln (50 g) (Product G-1210, Bachem) was dissolved in a 2 mol/l solution of sodium hydroxide (200 ml). To the resulting solution were added benzyloxycarbonyl chloride (51 g) and a 2 mol/l solution of sodium hydroxide (150 ml), followed by stirring at room temperature for 2 hours. To the resulting solution was added concentrated hydrochloric acid to adjust the solution to pH 2, whereby 85 g of benzyloxycarbonylated alanylglutamine (hereinafter referred to as Z-Ala-Gln) was obtained as crystals, which was then dried.

Then, the crystals of Z-Ala-Gln (35 g) were dissolved in an N,N-dimethylformamide-ethyl acetate solution (200 ml, 4:1). To the resulting solution were successively added N-hydroxysuccinimide (12 g), dicyclohexylcarbodiimide (22 g) and ethyl acetate (50 ml), followed by stirring at room temperature for 12 hours. After acetic acid (1 ml) was added, the resulting mixture was filtered, and the obtained filtrate was concentrated under reduced pressure to obtain 30 g of succinimidized Z-Ala-Gln (hereinafter referred to as Z-Ala-Gln-ONSu) as crystals.

The crystals were washed with isopropyl alcohol and then dried. The obtained crystals of Z-Ala-Gln-ONSu (23 g) were suspended in a 95% aqueous solution of methanol (300 ml), and palladium carbon (500 mg) was added thereto, followed by stirring at room temperature for 12 hours. To the resulting solution was added water (100 ml) and the mixture was filtered. The obtained filtrate was concentrated under reduced pressure to obtain crystals. The obtained crystals were suspended in a solution consisting of methanol (200 ml) and triethylamine (30 ml), followed by stirring at room temperature for 12 hours. The solution was filtered and the obtained crystals were washed with methanol and then dried to obtain 8 g of cyclo(Ala-Gln) as crystals.

Analysis and determination of the diketopiperazine and dipeptides were carried out in the following manner by using high performance liquid chromatography (HPLC).

The diketopiperazine was analyzed and determined by using Aminex HPX-87H ion-exchange column (7.8 mmID x 300 mm, Bio-Rad Laboratories Japan). As the mobile phase, a mixed solution of 5 mmol/l sulfuric acid and acetonitrile (7:3) was used. The HPLC conditions are as follows: flow rate of mobile phase, 0.6 ml/min; column temperature, 60°C; and detection wavelength, UV 250 nm.

The dipeptides were derivatized with 9-fluorenylmethoxycarbonyl (FMOC) and then analyzed by HPLC. Derivatization with FMOC was carried out by adding to a sample diluted with 100 mmol/l borate buffer (pH 9.0) an equal volume of a 6 mmol/l solution of 9-fluorenylmethyl chloroformate (FMOC-Cl) in acetone, and then allowing the resulting mixture to stand at room temperature for 40 minutes.

To the above mixture was added hexane (1.25 times volume), and after vigorous stirring, the upper hexane layer was removed. This procedure was repeated once more. To the remaining lower layer was added an equal volume of a mixture of 250 mmol/l borate buffer (pH 5.5) and acetonitrile (3:1). The resulting mixture was used as an FMOC-derivatized sample.

The FMOC-derivatized sample was analyzed and determined by using Develosil ODS-HG-3 column (4.6 mmID x 250 mm, Nomura Chemical Co., Ltd.). The HPLC analysis was carried out under the following conditions (1) or (2) according to the kind of dipeptides and impurities contained in the sample.

### Conditions (1)

The following mobile phases were used: mobile phase A in which the ratio of 20 mmol/l ammonium phosphate buffer (pH 6.5, adjusted with aqueous ammonia) to methanol is 17:3, and mobile phase B in which the ratio of acetonitrile to water is 9:1. The flow rate of the mobile phase was 1 ml/min, and the ratio of mobile phase A to mobile phase B was 82:18 at minute 0 and thereafter changed with a linear gradient so that the ratio became 1:99 after 30 minutes. Measurement was carried out by fluorescence detection at a column temperature of 40°C, at an excitation wavelength of 254 nm and at a detection wavelength of 630 nm.

### Conditions (2)

The following mobile phases were used: mobile phase C in which the ratio of a 6 ml/l solution of acetic acid (pH 3.6, adjusted with triethylamine) to acetonitrile is 8:2, and mobile phase D in which the ratio of a 6 ml/l solution of acetic acid (pH 3.6, adjusted with triethylamine) to acetonitrile is 3:7. The flow rate of the mobile phase was 1 ml/min, and the ratio of mobile phase C to mobile phase D was 85:15 at minute 0 and thereafter changed with a linear gradient so that the ratio became 0:100 after 25 minutes. Measurement was carried out by fluorescence detection at a column temperature of 40°C, at an excitation wavelength of 254 nm and at a detection wavelength of 630 nm.

### Best Modes for Carrying Out the Invention

### Example 1

Acquisition of Microorganisms Having the Ability To Produce a Dipeptide from a Diketopiperazine (1): Screening for Microorganisms Having the Ability To Assimilate a Diketopiperazine

Soil was sampled and ca. 2 g of the soil sample was suspended in 10 ml of sterile water. The suspension was gently shaken at room temperature for 30 minutes and allowed to stand for 10 minutes. The obtained supernatant (0.1 ml) was spread on agar medium A [2.5 g/l ammonium nitrate, 1 g/l disodium hydrogenphosphate, 0.5 g/l potassium dihydrogenphosphate, 0.5 g/l magnesium sulfate (MgSO₄·7H₂O), 0.1 g/l iron sulfate (FeSO₄·7H₂O), 0.1 g/l calcium chloride (CaCl₂·2H₂O), 0.88 mg/l zinc sulfate (ZnSO₄·7H₂O), 0.393 mg/l copper sulfate (CuSO₄·5H₂O), 0.072 mg/l manganese chloride (MnCl₂·4H₂O), 0.088 mg/l borax (Na₂B₄O₇·10H₂O), 0.037 mg/l ammonium molybdate [(NH₄)₆Mo₇O₂₄ · 4H₂O], 0.001 mg/l thiamine hydrochloride, 0.002 mg/l riboflavin, 0.002 mg/l calcium pantothenate, 0.002 mg/l pyridoxine hydrochloride, 0.0001 mg/l biotin, 0.001 mg/l p-aminobenzoic acid, 0.002 mg/l nicotinic acid and 15 g/l agar, pH 7.2] containing 2 g/l cyclo(Ala-Gln) as the sole carbon source, and cultured at 30°C for 2 days to form colonies. The formed colonies were spread on agar medium A containing 2 g/l cyclo(Ala-Gln) and agar medium A without cyclo(Ala-Gln), and cultured at 30°C for 2 days. Strains which grew on agar medium A containing cyclo(Ala-Gln) and did not grow on agar medium A without cyclo(Ala-Gln) were selected.

The strains selected above were spread on agar medium B [20 g/l ordinary bouillon medium (Kyokuto Pharmaceutical Industrial Co., Ltd.) and 15 g/l agar, pH 7.2] and cultured at 30°C for one day. Then, one platinum loop of each colony that appeared on the agar medium was inoculated into a 300-ml flask containing 40 ml of a medium prepared by adding 2 g/l cyclo(Ala-Gln) to liquid medium A [2 g/l glucose, 2 g/l ammonium nitrate, 1 g/l potassium dihydrogenphosphate, 3 g/l dipotassium hydrogenphosphate, 0.3 g/l magnesium sulfate (MgSO₄·7H₂O), 10 mg/l iron sulfate (FeSO₄·7H₂O), 10 mg/l manganese sulfate (MnSO₄·nH₂O), 0.037 mg/l ammonium molybdate [(NH₄)₆Mo₇O₂₄·4H₂O], 0.88 mg/l zinc sulfate (ZnSO₄·7H₂O), 0.393 mg/l copper sulfate (CuSO₄·5H₂O), 0.72 mg/l manganese chloride (MnCl₂·7H₂O), 0.088 mg/l borax (Na₂B₄O₇·10H₂O), 0.001 mg/l thiamine hydrochloride, 0.002 mg/l riboflavin, 0.002 mg/l calcium pantothenate, 0.002 mg/l pyridoxine hydrochloride, 0.0001 mg/l biotin, 0.001 mg/l p-aminobenzoic acid and 0.002 mg/l nicotinic acid], followed by culturing with shaking at 30°C for one day.

The culture supernatant was analyzed by HPLC to select strains giving a culture wherein cyclo(Ala-Gln) did not remain as the strains having the ability to assimilate cyclo(Ala-Gln).

### Example 2

### Acquisition of Microorganisms Having the Ability To Produce a Dipeptide from a Diketopiperazine (2): Screening for Microorganisms Having the Ability To Decompose Cyclo(Ala-Gln)

Each of the strains selected in Example 1 was spread on agar medium B and cultured at 30°C for one day. One platinum loop of the cultured cells was inoculated into a 300-ml flask containing 40 ml of liquid medium A comprising 2 g/l cyclo(Ala-Gln) and cultured with shaking at 30°C for one day. The resulting culture was centrifuged (8,000 rpm, 10 minutes) to precipitate cells. The cells were suspended in a 0.85% aqueous solution of sodium chloride, followed by centrifugation (8,000 rpm, 10 minutes). After this procedure was repeated once more, the obtained cells were frozen at -80°C for one hour.

The frozen cells were thawed at an ordinary temperature and then suspended in 50 mmol/l potassium phosphate buffer (pH 7.5) to a concentration of ca. 20 g/l in terms of wet cell weight. The suspension (0.9 ml) and 0.1 ml of 50 mmol/l potassium phosphate buffer (pH 7.5) containing 10 g/l cyclo(Ala-Gln) were put into a 2-ml plastic tube and gently shaken at 30°C for 8 hours. The reaction mixture was centrifuged (10,000 rpm, 10 minutes), and the obtained supernatant was analyzed by HPLC.

As a result, the decrease in cyclo(Ala-Gln) was observed in many reaction mixtures. For example, ca. 0.5 g/l cyclo(Ala-Gln) disappeared in 8 hours from the reaction mixtures respectively containing Microbacterium luteolum No. 93, Microbacterium sp. No. 119, Sinorhizobium sp. No. 1, Sinorhizobium sp. No. 164, Pseudomonas sp. No. 107 and Pseudomonas sp. No. 108.

The above strains were regarded as candidates for the strains having the ability to produce a dipeptide from a diketopiperazine wherein two kinds of α-amino acids are condensed with each other.

### Example 3

Activity To Decompose a Diketopiperazine Wherein Glycine and Glutamine Are Condensed with Each Other [Hereinafter Referred to as Cyclo(Gly-Gln)]

The suspension of the cells of Microbacterium luteolum No. 93 which underwent freezing and thawing prepared in Example 2 (0.9 ml) and 0.1 ml of 50 mmol/l potassium phosphate buffer (pH 7.5) containing 5 g/l cyclo(Gly-Gln) (Bachem) were put into a 2-ml plastic tube and gently shaken at 30°C for 8 hours. The reaction mixture was centrifuged (10,000 rpm, 10 minutes), and the obtained supernatant was analyzed by HPLC. As a result, it was revealed that ca. 0.1 g/l cyclo(Gly-Gln) disappeared in 8 hours.

### Example 4

### Activity To Decompose a Diketopiperazine Wherein Leucine and Phenylalanine Are Condensed with Each Other [Hereinafter Referred to as Cyclo(Leu-Phe)]

The suspension of the cells of Microbacterium luteolum No. 93 which underwent freezing and thawing prepared in Example 2 (0.9 ml) and 0.1 ml of a 70% aqueous solution of ethanol containing 300 mg/l cyclo(Leu-Phe) (Bachem) were put into a 2-ml plastic tube and gently shaken at 30°C for 24 hours. The reaction mixture was centrifuged (10,000 rpm, 10 minutes), and the obtained supernatant was analyzed by HPLC. As a result, it was revealed that ca. 10 mg/l cyclo(Leu-Phe) disappeared in 24 hours.

### Example 5

### Production of Ala-Gln

To each of the suspensions of the cells of Microbacterium luteolum No. 93 and Microbacterium sp. No. 119 which underwent freezing and thawing prepared in Example 2 (0.9 ml) were added cyclo(Ala-Gln) and cobalt chloride to give the final concentrations of 2 g/l and 1 mmol/l, respectively. Each mixture (1 ml) was put into a 2-ml plastic tube and gently shaken at 30°C for 2 hours. The reaction mixture was centrifuged (10,000 rpm, 10 minutes), and the obtained supernatant was subjected to FMOC derivatization and then analyzed by HPLC. The results are shown in Table 1.

**Table 1**

| Strain | Alanylglutamine (mg/l) | Glutamylalanine (mg/l) |
|---|---|---|
| Microbacterium luteolum No. 93 | 37 | ND |
| Microbacterium sp. No. 119 | 29 | ND |

The proportions of alanylglutamine in the dipeptides formed from cyclo(Ala-Gln) by Microbacterium luteolum No. 93 and Microbacterium sp. No. 119 were both 100%.

### Example 6

### Production of Glutamylalanine (Gln-Ala)

To each of the suspensions of the cells of Sinorhizobium sp. No. 1, Sinorhizobium sp. No. 164, Pseudomonas sp. No. 107 and Pseudomonas sp. No. 108 which underwent freezing and thawing prepared in Example 2 (0.9 ml) were added cyclo(Ala-Gln) and EDTA to give the final concentrations of 2 g/l and 10 mmol/l, respectively. Each mixture (1 ml) was put into a 2-ml plastic tube and gently shaken at 30°C for 2 hours. The reaction mixture was centrifuged (10,000 rpm, 10 minutes) to precipitate the cells, and the obtained supernatant was subjected to FMOC derivatization and then analyzed by HPLC. The results are shown in Table 2.

**Table 2**

| Strain | Alanylglutamine (mg/l) | Glutamylalanine (mg/l) |
|---|---|---|
| Sinorhizobium sp. No. 1 | 34 | 92 |
| Sinorhizobium sp. No. 164 | 27 | 187 |
| Pseudomonas sp. No. 107 | 11 | 163 |
| Pseudomonas sp. No. 108 | 11 | 150 |

The proportions of glutamylalanine in the dipeptides formed from cyclo(Ala-Gln) by Sinorhizobium sp. No. 1, Sinorhizobium sp. No. 164, Pseudomonas sp. No. 107 and Pseudomonas sp. No. 108 were 73%, 87%, 94% and 92%, respectively.

## Claims

1. A process for producing a dipeptide, which comprises:
allowing an enzyme source and a diketopiperazine wherein one or two kinds of α-amino acids or derivatives thereof are condensed with each other to be present in an aqueous medium, said enzyme source being a culture of a microorganism having the ability to produce a dipeptide from a diketopiperazine wherein two kinds of α-amino acids are condensed with each other or a treated matter of the culture;
allowing the dipeptide to form and accumulate in the aqueous medium; and
recovering the dipeptide from the aqueous medium (provided that the case in which the diketopiperazine is a diketopiperazine wherein aspartic acid and phenylalanine are condensed with each other and the dipeptide is aspartylphenylalanine is excluded).

2. The process according to Claim 1, wherein the microorganism having the ability to produce a dipeptide from a diketopiperazine wherein two kinds of α-amino acids are condensed with each other is a microorganism which produces dipeptides in which the proportion of one kind of dipeptide is 70% or more.

3. The process according to Claim 1 or 2, wherein the microorganism having the ability to produce a dipeptide from a diketopiperazine wherein two kinds of α-amino acids are condensed with each other is a microorganism obtained by a method comprising:
[1] the step of culturing test microorganisms using a medium comprising a diketopiperazine wherein two kinds of α-amino acids are condensed with each other as the sole carbon source or nitrogen source;
[2] the step of selecting microorganisms which are recognized to grow in the above step [1]; and
[3] the step of selecting a microorganism which forms and accumulates a dipeptide in an aqueous medium when the diketopiperazine used in the above step [1] and the microorganisms selected in the above step [2] are allowed to be present in the aqueous medium.

4. The process according to Claim 2, wherein the microorganism having the ability to produce a dipeptide from a diketopiperazine wherein two kinds of α-amino acids are condensed with each other is a microorganism obtained by a method comprising:
[1] the step of culturing test microorganisms using a medium comprising a diketopiperazine wherein two kinds of α-amino acids are condensed with each other as the sole carbon source or nitrogen source;
[2] the step of selecting microorganisms which are recognized to grow in the above step [1]; and
[3] the step of selecting a microorganism which forms and accumulates dipeptides in an aqueous medium, the proportion of one kind of dipeptide in the dipeptides formed and accumulated being 70% or more, when the diketopiperazine used in the above step [1] and the microorganisms selected in the above step [2] are allowed to be present in the aqueous medium.

5. The process according to any one of Claims 1 to 4, wherein the microorganism having the ability to produce a dipeptide from a diketopiperazine wherein two kinds of α-amino acids are condensed with each other is a microorganism belonging to the genus Microbacterium, Sinorhizobium or Pseudomonas.

6. The process according to Claim 5, wherein the microorganism belonging to the genus Microbacterium is Microbacterium luteolum.

7. A process for producing a dipeptide, which comprises:
allowing an enzyme source and a diketopiperazine wherein one or two kinds of α-amino acids or derivatives thereof are condensed with each other to be present in an aqueous medium, said enzyme source being a culture of a microorganism belonging to the genus Microbacterium, Sinorhizobium or Pseudomonas having the ability to produce a dipeptide from a diketopiperazine wherein two kinds of α-amino acids are condensed with each other or a treated matter of the culture;
allowing the dipeptide to form and accumulate in the aqueous medium; and
recovering the dipeptide from the aqueous medium.

8. The process according to Claim 8, wherein the microorganism belonging to the genus Microbacterium is Microbacterium luteolum.

9. The process according to any one of Claims 1 to 8, wherein the α-amino acid is an α-amino acid selected from the group consisting of alanine, glutamine, glutamic acid, glycine, valine, leucine, isoleucine, proline, phenylalanine, tryptophan, methionine, serine, threonine, cysteine, asparagine, tyrosine, lysine, arginine, histidine, aspartic acid and ornithine.

10. The process according to any one of Claims 1 to 9, wherein the two kinds of α-amino acids are alanine and glutamine, and the dipeptide is alanylglutamine.

11. The process according to any one of Claims 1 to 10, wherein the treated matter of the culture is concentrated culture, dried culture, cells obtained by centrifuging the culture, or a product obtained by subjecting the cells to drying, freeze-drying, treatment with a surfactant, treatment with a solvent, enzymatic treatment, immobilization, mechanical friction or ultrasonication.

12. A microorganism having the ability to produce a dipeptide from a diketopiperazine wherein two kinds of α-amino acids are condensed with each other which is selected from the group consisting of Microbacterium luteolum No. 93 (FERM BP-08513), Microbacterium sp. No. 119 (FERM BP-08514), Sinorhizobium sp. No. 1 (FERM BP-08509), Sinorhizobium sp. No. 164 (FERM BP-08510), Pseudomonas sp. No. 107 (FERM BP-08511) and Pseudomonas sp. No. 108 (FERM BP-08512).
